# EUROPÄISCHE PATENTANMELDUNG

(11) **EP 0 690 056 A2**
(43) Veröffentlichungstag der Anmeldung: **03.01.1996**
(21) Anmeldenummer: 95110018.9
(22) Anmeldetag: 27.06.1995
(51) Int. Cl.: C07D 473/18, C07D 473/34, C07D 473/26

(54) **Verfahren zur Herstellung von Purinen**

(30) Priorität: 28.06.1994 DE 4422587
(71) Anmelder: SKW Trostberg Aktiengesellschaft, D-83308 Trostberg (DE)
(72) Erfinder: Thalhammer, Franz, Dr., D-83308 Trostberg (DE); Graefe, Jürgen, Dr., D-83308 Trostberg (DE)
(74) Vertreter: Huber, Bernhard, Dipl.-Chem.

(57) **Zusammenfassung**

Zur Herstellung eines Purins der allgemeinen Formel (I), 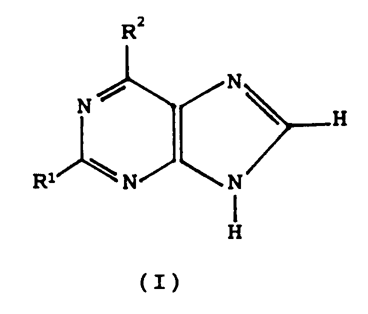
in der R¹ und R² gleich oder verschieden sein können und H, OH, SH, NH₂, N-(Di)-Alkyl, Halogen, O-Alkyl, S-Alkyl, Alkyl oder Aryl bedeuten und Alkyl einen aliphatischen Rest mit 1 bis 4 Kohlenstoffatomen und Aryl einen gegebenenfalls mit CH₃, OH, NH₂, Halogen-substituierten Phenylrest darstellt, aus dem entsprechenden 4-Amino-5-nitrosopyrimidin der Formel (II), 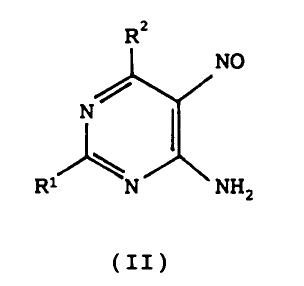
in der R¹ und R² die oben angegebene Bedeutung haben, formyliert man die Verbindung der Formel (II) in Gegenwart von Ameisensäure und einem Katalysator auf Basis eines Edelmetalls in einem Lösemittel bei einer Temperatur von 80 bis 220°C reduktiv und cyclisiert das intermediär gebildete 4-Amino-5-formylaminopyrimidin.

## Beschreibung

Die Erfindung betrifft ein Verfahren zur Herstellung von Purinen aus den entsprechenden 4-Amino-5-nitrosopyrimidinen durch reduktive Formylierung und anschließende Cyclisierung der intermediär entstehenden 4-Amino-5-formylaminopyrimidine.

Purine werden bisher überwiegend aus Salzen von 4,5-Diaminopyrimidinen oder aus 4-Amino-5-(formylamino)pyrimidinen hergestellt. Beispielsweise erhält man gemäß der EP-A 415 028 Guanin durch Erhitzen von 2,4,5-Triamino-6-hydroxypyrimidinsulfat (TAHP-Sulfat) mit Natriumformiat in Ameisensäure.

Entsprechend der DE-PS 37 29 471 wird Guanin durch Erhitzen von TAHP-Sulfat in Formamid erhalten, wobei Ammoniak als Zersetzungsprodukt von Formamid das Sulfat neutralisiert. Diese Verfahren haben den Nachteil, daß sie als Ausgangsstoffe 4,5-Diaminopyrimidine benötigen, die durch eine katalytische Hydrierung mit Wasserstoff aus den entsprechenden 5-Nitrosopyrimidinen, beispielsweise analog DE-PS 36 38 635, hergestellt werden, wozu teure Autoklaven oder Schleifenreaktoren nötig sind. Darüber hinaus müssen die instabilen 4,5-Diaminopyrimidine zur Stabilisierung in die Form der Sulfate gebracht werden. Dies führt zu einem hohen Salzanfall, der sich im Hinblick auf Wirtschaftlichkeit und Ökobilanz nachteilig auswirkt.

In der DE-PS 36 38 635 wird die Herstellung von 2,4-Diamino-5-formylamino-6-hydroxypyrimidin (DAFHP) durch katalytische Hydrierung der entsprechenden Nitrosoverbindungen mit Wasserstoff und anschließende Formylierung beschrieben. DAFHP kann gemäß DE-OS 41 36 114 in Formamid zu Guanin umgesetzt werden. Bei allen diesen Verfahren ist eine Isolierung und gegebenenfalls Reinigung der Vorstufe nötig, weshalb Purine demnach in zwei getrennten Verfahrensschritten aus den entsprechenden 5-Nitrosopyrimidinen hergestellt werden müssen.

Schließlich ist aus der EP-A 304 004 ein Eintopfverfahren zur Herstellung der Natriumsalze von Purinen bekannt. Nach diesem Verfahren werden in Formamid aus 4-Aminopyrimidinen durch Nitrosierung 4-Amino-5-nitrosopyrimidine hergestellt, diese mit Natriumdithionit reduziert, nachfolgend mit Ameisensäure formyliert und schließlich bei erhöhten Temperaturen zu den entsprechenden Purinen cyclisiert. Dieses Verfahren hat den Nachteil, daß infolge der Verwendung von Natriumdithionit schwefelhaltige Salze anfallen und darüber hinaus SO₂ in großen Mengen freigesetzt wird. Vom ökologischen Standpunkt ist dieses Verfahren großtechnisch deshalb nicht vertretbar.

Der vorliegenden Erfindung liegt daher die Aufgabe zugrunde, ein Verfahren zur Herstellung von Purinen der allgemeinen Formel (I), 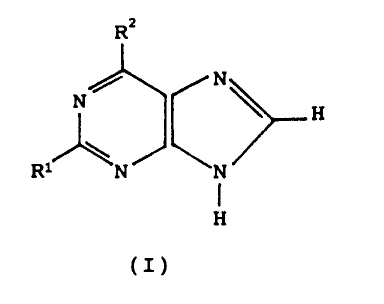
in der R¹ und R² gleich oder verschieden sein können und H, OH, SH, NH₂, N-(Di)-Alkyl, Halogen, O-Alkyl, S-Alkyl, Alkyl oder Aryl bedeuten und Alkyl einen aliphatischen Rest mit 1 bis 4 Kohlenstoffatomen und Aryl einen gegebenenfalls mit CH₃, OH, NH₂, Halogen-substituierten Phenylrest darstellt, aus den entsprechenden 4-Amino-5-nitrosopyrimidinen zu schaffen, welches die genannten Nachteile des Standes der Technik nicht aufweist, sondern ohne großen technischen Aufwand und insbesondere ohne Zuhilfenahme von elementarem Wasserstoff oder des ökologisch bedenklichen Natriumdithionits eine problemlose Herstellung der gewünschten Purine ermöglicht.

Diese Aufgabe wird erfindungsgemäß dadurch gelöst, daß man ein 4-Amino-5-nitrosopyrimidin der allgemeinen Formel (II) in der R¹ und R² die oben angegebene Bedeutung haben, welches dadurch gekennzeichnet ist, daß man die Verbindung der Formel (II) in Gegenwart von Ameisensäure und einem Katalysator auf Basis eines Edelmetalls in einem Lösemittel bei einer Temperatur von 80 bis 220°C reduktiv formyliert und das intermediär gebildete 4-Amino-5-formylaminopyrimidin cyclisiert.

Es hat sich überraschenderweise gezeigt, daß man erfindungsgemäß die Purine der allgemeinen Formel (I) in einem Eintopfverfahren mit hohen Ausbeuten und guten Reinheiten erhalten kann, wobei Ameisensäure gleichzeitig als Reduktions- und Formylierungs-Reagens und darüber hinaus gegebenenfalls noch als Lösungsmittel fungiert. Außerdem muß der Edelmetall-Katalysator vor der Ringschlußreaktion zu den Purinen nicht abgetrennt werden, was ebenfalls nicht vorhersehbar war.

Beim Verfahren der Erfindung wird ein 4-Amino-5-nitrosopyrimidin der allgemeinen Formel (II) eingesetzt, 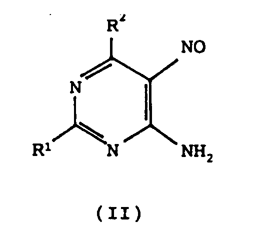
in der die Reste R¹ und R² gleich oder verschieden sind und H, OH, SH, NH₂, N-(Di)-Alkyl, Halogen, O-Alkyl, S-Alkyl, Alkyl sowie Aryl bedeuten, Alkyl einen aliphatischen Rest mit 1 bis 4 Kohlenstoffatomen darstellt. Diese Ausgangsverbindung wird in einem Lösemittel suspendiert, welches vorzugsweise aus Ameisensäure und/oder Formamid besteht. Gemäß einer bevorzugten Ausführungsform wird das 4-Amino-5-nitrosopyrimidin nach seiner Herstellung durch Nitrosierung von 4-Aminopyrimidinen in wasserfeuchter Form bzw. bereits in der anfallenden Reaktionssuspension eingesetzt.

Die Konzentration der Nitroso-Verbindung der Formel (II) im Lösemittel ist in weiten Grenzen variierbar und beträgt 0,1 bis 3,0, vorzugsweise 1,0 bis 2,0 mol pro Liter Solvens. Wird in Formamid als Lösemittel gearbeitet, muß dem Reaktionsgemisch Ameisensäure zugesetzt werden, und zwar insbesondere 2,0 bis 10,0 mol, vorzugsweise 3,5 bis 5,0 mol Ameisensäure pro Mol Nitroso-Verbindung der Formel (II). Alternativ kann Ameisensäure selbst gegebenenfalls in wäßriger Verdünnung bis 20 Gew.-% als Lösemittel verwendet werden.

Zur Beschleunigung der Reaktion und Verbesserung der Rührbarkeit bei höher konzentrierten Ansätzen kann der Reaktionsmischung ein Salz der Ameisensäure, insbesondere ein Formiat der Alkali- und Erdalkalimetalle, von Ammoniak oder Aminen zugegeben werden. Bevorzugt sind hierbei Natrium-, Ammonium- und Triethylammoniumformiat, wobei ein Molverhältnis von 0,1 bis 5, besonders bevorzugt etwa 0,5, bezogen auf die eingesetzte Nitroso-Verbindung der Formel (II) verwendet wird. Die Zugabe des Formiats kann in fester oder gelöster Form erfolgen.

Der auf diese Weise hergestellten Reaktionsmischung wird ein Edelmetall-Katalysator zugesetzt. Bevorzugt wird ein Katalysator auf Basis Palladium, Platin, Ruthenium oder Rhodium, vorzugsweise auf einem Trägermaterial, insbesondere auf Aktivkohle, zugesetzt. Handelsübliche Produkte sind geeignet. Der Edelmetallgehalt des Katalysators beträgt zweckmäßig 0,1 bis 10 Gew.-%. Als besonders geeignet hat sich ein Katalysator mit etwa 5 Gew.-% Palladium auf Aktivkohle erwiesen. Der Katalysator wird vorzugsweise in einer solchen Menge verwendet, daß der Anteil des reinen Edelmetalls 20 bis 2000 mg, besonders bevorzugt 100 bis 500 mg pro Kilogramm eingesetzter Nitroso-Verbindung der Formel (II), beträgt.

Nach der Zugabe des Katalysators tritt unter Rühren bereits bei Raumtemperatur eine langsame Freisetzung von CO₂ ein. Anschließend wird die gewünschte Reaktionstemperatur eingestellt. Vorzugsweise wird während einer ersten Periode von 1 bis 4 Stunden die Temperatur auf 80 bis 140°C, insbesondere 100 bis 120°C eingestellt. Während dieser Zeit entfärbt sich die anfangs intensiv rot bis violett gefärbte Suspension. Anschließend wird die Temperatur bei Verwendung von Formamid als Lösemittel vorzugsweise auf 150 bis 190°C erhöht, wobei gegebenenfalls entstehendes Reaktionswasser destillativ abgetrennt wird. Bei Verwendung von Ameisensäure als Lösemittel wird die Temperatur vorzugsweise auf 100 bis 170°C erhöht. Temperaturen über ca. 110°C erfordern hierbei die Anwendung von Drücken bis ca. 20 bar. Es ist auch möglich, höhere Temperaturen durch Zumischen hochsiedender Lösemittel wie z.B. Glykolether, Diphenylether etc. zu erreichen. Dies erfordert allerdings eine aufwendige destillative Rückgewinnung des betreffenden Lösemittels am Ende der Herstellung des jeweiligen Purins.

Die Dauer des Erhitzens ist abhängig von der Höhe der Reaktionstemperatur. In Formamid sind ca. 10 Stunden bei 160°C oder ca. 3 Stunden bei 180°C nötig. Bei Verwendung von Ameisensäure beträgt die Reaktionsdauer ca. 20 Stunden bei 110°C oder ca. 2 Stunden bei 160°C unter einem Druck von 8 bar.

Die so erhaltene Suspension des Purins enthält keine nachweisbaren Mengen der Ausgangsverbindung oder des 4-Amino-5-formylaminopyrimidins. Allerdings liegt das Purin teilweise als Formiat vor. Ebenso sind noch die gegebenenfalls zugesetzten Ameisensäuresalze und der Edelmetall-Katalysator sowie in geringen Mengen gefärbte Zersetzungsprodukte enthalten.

Für die Isolierung des Purins und die Rückgewinnung des Edelmetall-Katalysators wird die Suspension zweckmäßigerweise abgekühlt und filtriert. Zur besseren Filtrierbarkeit bei sehr viskosen Suspensionen kann Wasser oder ein organisches Lösemittel zugesetzt werden, das sich durch Destillation wieder leicht aus der Mutterlauge abtrennen läßt, z.B. Aceton, Methanol oder Acetonitril.

Bei dieser Arbeitsweise wird das Purin gemeinsam mit dem Edelmetall-Katalysator abfiltriert und mit Wasser gewaschen. Der Filterkuchen kann dann in Natronlauge mit einem Gehalt von 5 bis 10 Gew.-% aufgenommen werden, wobei der Katalysator ungelöst zurückbleibt und durch Filtration abgetrennt werden kann.

Vorteilhafterweise erhält man so den eingesetzten Edelmetall-Katalysator nahezu quantitativ zurück, was für ein wirtschaftliches Verfahren wegen des hohen Preises der Edelmetalle sinnvoll ist. Der Katalysator behält auch überraschenderweise trotz der extremen Temperaturbelastung weitgehend seine Aktivität, so daß er in Folgeansätzen wieder eingesetzt werden kann.

Zur Abtrennung von Nebenprodukten kann das Filtrat der Katalysatorentfernung bei einer Temperatur von 5 bis 80°C mit 5 bis 50 Gew.-% Aktivkohle, bezogen auf das Gewicht des eingesetzten Filterkuchens, versetzt und 20 bis 60 Minuten gerührt werden. Für besondere Reinheitsanforderungen kann diese Prozedur ein oder mehrmals wiederholt werden. Anschließend wird zur Freisetzung des Purins aus seinem Natriumsalz durch kontrollierte Zugabe einer Säure, beispielsweise Ameisensäure, Schwefelsäure oder Kohlensäure, ein pH-Wert von 8 bis 10 eingestellt, wobei das Purin aus der wäßrigen Lösung ausfällt und durch Filtration isoliert werden kann. Alternativ kann die Abtrenung des Purins und die Rückgewinnung des Katalysators durchgeführt werden, indem man die erhaltene Reaktionssuspension, vorteilhafterweise unter Vakuum, bis zur Trockene eindampft und den Eindampfrückstand in gleicher Weise weiterbehandelt, wie es oben für den Filtrationsrückstand beschrieben wird.

Durch die Rückgewinnung des Katalysators während der Reinigung des Purins läßt sich gegenüber dem Stand der Technik der Salzanfall deutlich reduzieren.

Die Ausbeuten nach dem erfindungsgemäßen Verfahren erreichen Werte von bis zu 94 % d. Th., wobei die Purine in Reinheiten von über 98 % anfallen. Aufgrund dieser hohen Ausbeuten, des reduzierten Salzanfalls gegenüber dem Stand der Technik sowie des geringen technischen Aufwands eignet sich das erfindungsgemäße Verfahren besonders gut für die Durchführung im technischen Maßstab.

Die nachstehenden Beispiele sollen die Erfindung näher erläutern.

### Beispiel 1

31,6 g (0,2 mol) 2,4-Diamino-6-hydroxy-5-nitrosopyrimidin (DAHNP) wurden in 150 ml Formamid und 43,3 g (0,8 mol) 85 %-iger Ameisensäure suspendiert. Unter kräftigem Rühren wurden dann 1,0 g Palladium-Katalysator auf Aktivkohle (Typ E10 R/W 5 % Pd, 50 % feucht) zugesetzt, wobei eine leichte Gasentwicklung einsetzte. Die Temperatur wurde auf 110°C eingestellt und 3 Stunden gehalten. Die anfangs pinkfarbene Suspension war nach ca. 2 Stunden grau geworden. Anschließend wurde 3 Stunden bei 180°C erhitzt. Die enstandene graue Suspension wurde auf 20°C gekühlt, der Feststoff über eine Filternutsche abgesaugt, dreimal mit 50 ml Wasser gewaschen und in 320 ml einer 5 Gew.-%-igen Natronlauge eingerührt. Der Edelmetall-Katalysator wurde von der Lösung des Natriumsalzes des Guanins abfiltriert und zweimal mit 5 ml Wasser gewaschen. Die vereinigten Filtrate wurden auf 50°C erwärmt und mit 3 g Aktivkohle versetzt. Nach 30 Minuten wurde diese abfiltriert und das Guanin durch langsame Zugabe von 85 %-iger Ameisensäure bis pH 9,5 ausgefällt. Das Produkt wurde durch Filtration isoliert, zweimal mit 50 ml Wasser gewaschen und bei 80°C im Vakuum getrocknet.

Es wurden 28,8 g (0,188 mol, 94 % d. Th.) Guanin mit geringem Gelbstich erhalten, das nach HPLC einen Gehalt von 98,5 % aufwies. In der Mutterlauge wurde kein gelöstes Palladium gefunden.

### Beispiel 2

Entsprechend Beispiel 1 wurden 31,6 g (0,2 mol) DAHNP unter Verwendung des im Beispiel 1 zurückgewonnenen Edelmetall-Katalysators, der mit 15 Gew.-% frischem Katalysator ergänzt worden war, zu Guanin umgesetzt. Es wurden 28,2 g (0,184 mol, 92 % d. Th.) in Form eines Pulvers mit leichtem Gelbstich erhalten.

### Beispiel 3

845 g filterfeuchtes DAHNP mit einer Restfeuchte von 45 % (3,0 mol) wurden in 2,5 l Formamid suspendiert und mit 650 g (12,0 mol) 85 %-iger Ameisensäure versetzt. Nach Zugabe von 15 g eines Palladium-Katalysators auf Aktivkohle (analog Beispiel 1) wurde die Temperatur auf 110 bis 120°C erhöht, wobei eine kräftige Entwicklung von CO₂ zu beobachten war. Nach etwa 1 Stunde war die anfangs rosafarbene Suspension grau. Es wurde weitere 30 Minuten bei 110°C gerührt und dann ca. 600 ml Flüssigkeit abdestilliert, bis die Temperatur des Reaktionsgemisches 180°C erreicht hatte. Bei dieser Temperatur wurde dann 3 Stunden unter Rückfluß erhitzt. Nach Kühlung auf 60°C wurde 1 l einer Mischung von Methanol und Wasser im Verhältnis 1:1 zugegeben und nachfolgend auf 20°C gekühlt. Der enthaltene Feststoff wurde abgesaugt, zweimal mit jeweils 500 ml Wasser gewaschen und in 2,5 l 10 %-iger Natronlauge gelöst. Durch Filtration wurde der unlösliche Katalysator abgetrennt. Das Filtrat wurde mit 40 g einer handelsüblichen Aktivkohle versetzt und 30 Minuten bei 60°C gerührt. Nach dem Abtrennen der Aktivkohle durch Filtration wurde in die Lösung CO₂ eingeleitet, bis ein pH-Wert von 9,5 erreicht war. Dabei fiel ein farbloser Feststoff aus, der abfiltriert, zweimal mit jeweils 500 ml Wasser gewaschen und bei 60°C im Vakuum getrocknet wurde. Auf diese Weise wurden 426 g (2,82 mol, 94 % d. Th.) Guanin als fast farbloses Pulver erhalten.

### Beispiel 4

29,0 g (0,2 mol) 2,4-Diamino-6-hydroxypyrimidin-hydrat wurden in 150 ml Formamid mit 13,8 (0,2 mol) Natriumnitrit und 10,8 g (0,2 mol) 85 %-iger Ameisensäure nitrosiert und die erhaltene rosafarbene Suspension mit weiteren 42,4 g (0,8 mol) 85 %-iger Ameisensäure versetzt. Nach dem Einrühren von 1,0 g eines Palladium-Katalysators auf Aktivkohle entsprechend Beispiel 1 wurde 3 Stunden auf 120°C erhitzt. Die Suspension entfärbte sich dabei und wurde anschließend unter Abdestillieren einer kleinen Menge Wasser 3 Stunden lang auf 180°C erhitzt. Nach dem Abkühlen auf 80°C wurden 100 ml Wasser zugesetzt und der Feststoff abgesaugt. Der Filterkuchen wurde zweimal mit 50 ml Wasser gewaschen und in 320 ml einer 5 %-igen Natronlauge gelöst. Der unlösliche Edelmetall-Katalysator wurde abfiltriert und das Filtrat mit 3 g Aktivkohle bei 50°C 30 min. lang gerührt. Nach der Abtrennung der Aktivkohle wurde Guanin durch Zugabe von 20 %-iger Schwefelsäure bis pH 9,0 ausgefällt, abgesaugt, zweimal mit 50 ml Wasser gewaschen und bei 80°C im Vakuum getrocknet.

Auf diese Weise wurden 26,7 g (0,174 mol, 87 % d. Th.) 98,4 % reines Guanin erhalten.

### Beispiel 5

31,6 g (0,2 mol) DAHNP wurden mit 6,3 g (0,1 mol) Ammoniumformiat und 1,0 g Palladium-Katalysator analog Beispiel 1 in 150 ml 85 %-iger Ameisensäure suspendiert und 20 Stunden zum RückfluB erhitzt. Dabei entfärbte sich die Suspension nach ca. 3 Stunden. Anschließend wurde unter Vakuum zur Trockene eingedampft und der Rückstand in 350 ml 5 %-iger Natronlauge gelöst. Nach Abtrennen des Katalysators und Behandlung des Filtrats wie in Beispiel 1 wurden 26,5 g (0,170 mol, 85 % d. Th.) 97 %-iges Guanin erhalten, das noch ca. 1 % 2,4-Diamino-5-formylamino-6-hydroxypyrimidin enthielt.

### Beispiel 6

Eine Mischung aus 31,6 g (0,2 mol) DAHNP, 6,8 g (0,1 mol) Natriumformiat und 1,0 g Palladium-Katalysator entsprechend Beispiel 1 wurde in 150 ml 85 %-iger Ameisensäure 4 Stunden zum RückfluB erhitzt. Anschließend wurde die Suspension in einen Rührautoklaven eingefüllt und 3 Stunden auf 150°C erhitzt. Dabei bildet sich ein Innendruck von 6 bis 7 bar. Nach dem Abkühlen wurde entspannt, der Inhalt des Autoklaven entnommen und der Feststoff abgesaugt. Der Filterkuchen wurde analog Beispiel 1 weiterbehandelt. Es wurden 21,6 g (14,3 mol, 71 % d. Th.) Guanin mit einer Reinheit von 99,4 % erhalten. Durch Zugabe von Natronlauge zum Rückstand nach dem Eindampfen des Filtrats konnten weitere 6,2 g (20 % d. Th.) Guanin erhalten werden.

### Beispiel 7

Analog Beispiel 1 wurde unter Verwendung von 1,0 g Platin auf Aktivkohle (5 % Pt, 50 % feucht) aus 31,6 g (0,2 mol) DAHNP 28,9 g (0,189 mol, 94 % d. Th.) Guanin mit einer Reinheit von 98,9 % erhalten.

### Beispiel 8

13,9 g (0,1 mol) 4,6-Diamino-5-nitrosopyrimidin wurden in 50 ml Formamid suspendiert, 6,8 g (0,1 mol) Natriumformiat, 27,0 g (0,5 mol) 85 %-ige Ameisensäure und 0,5 g Palladium-Katalysator entsprechend Beispiel 1 zugegeben und die Mischung 4 Stunden auf 110 °C erhitzt. Die entfärbte Suspension wurde anschließend 3 Stunden auf 180°C aufgeheizt und dann abgekühlt. Zur besseren Verarbeitung wurden 50 ml Wasser zugesetzt und der Feststoff abfiltriert. Der Filterkuchen wurde in 160 ml 5 %-iger Natronlauge gelöst und der unlösliche Pd-Katalysator abgetrennt. Das klare Filtrat wurde nach einer 30-minütigen Behandlung mit 3 g Aktivkohle bei 50°C mit Schwefelsäure auf einen pH-Wert von 8,5 eingestellt. Dabei fiel ein farbloser Niederschlag von Adenin aus, der abgesaugt, zweimal mit 30 ml Wasser gewaschen und im Vakuum bei 80°C getrocknet wurde. Auf diese Weise wurden 12,2 g (0,090 mol, 90 % d. Th.) Adenin als weißes Pulver erhalten.

### Beispiel 9

Analog Beispiel 8 wurden 15,4 (0,1 mol) 2,4,6-Triamino-5-nitrosopyrimidin zu 2,6-Diaminopurin umgesetzt. Dabei wurden 12,2 g (0,081 mol, 81 % d. Th.) in Form eines leicht beigen Pulvers erhalten.

## Patentansprüche

1. Verfahren zur Herstellung eines Purins der allgemeinen Formel (I), 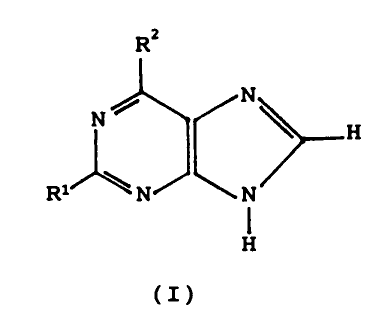 in der R¹ und R² gleich oder verschieden sein können und H, OH, SH, NH₂, N-(Di)-Alkyl, Halogen, O-Alkyl, S-Alkyl, Alkyl oder Aryl bedeuten und Alkyl einen aliphatischen Rest mit 1 bis 4 Kohlenstoffatomen und Aryl einen gegebenenfalls mit CH₃, OH, NH₂, Halogen-substituierten Phenylrest darstellt, aus dem entsprechenden 4-Amino-5-nitrosopyrimidin der Formel (II), 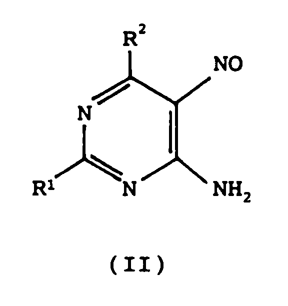 in der R¹ und R² die oben angegebene Bedeutung haben,
**dadurch gekennzeichnet,**
daß man die Verbindung der Formel (II) in Gegenwart von Ameisensäure und einem Katalysator auf Basis eines Edelmetalls in einem Lösemittel bei einer Temperatur von 80 bis 220°C reduktiv formyliert und das intermediär gebildete 4-Amino-5-formylaminopyrimidin cyclisiert.

2. Verfahren nach Anspruch 1,
**dadurch gekennzeichnet,**
daß als Lösemittel Formamid und/oder Ameisensäure verwendet wird.

3. Verfahren nach den Ansprüchen 1 oder 2,
**dadurch gekennzeichnet,**
daß die Konzentration der Verbindung der Formel (II) 0,1 bis 3,0 mol l⁻¹, vorzugsweise 1,0 bis 2,0 mol l⁻¹, beträgt.

4. Verfahren nach einem der Ansprüche 1 bis 3,
**dadurch gekennzeichnet,**
daß die Verbindung der Formel (II) in wasserfeuchter Form, insbesondere in einer nach einer vorangehenden Nitrosierung anfallenden und Natriumformiat enthaltenen Suspension, eingesetzt wird.

5. Verfahren nach einem der Ansprüche 2 bis 4,
**dadurch gekennzeichnet,**
daß man bei Verwendung von Formamid als Lösemittel die Ameisensäure in einem Molverhältnis von 2,0 bis 10,0, bevorzugt 3,5 bis 5,0, bezogen auf eingesetzte Verbindung der Formel (II) zusetzt.

6. Verfahren nach einem der Ansprüche 1 bis 5,
**dadurch gekennzeichnet,**
daß man wäßrige Ameisensäure mit einer Verdünnung bis 20 Gew.-% verwendet.

7. Verfahren nach einem der Ansprüche 1 bis 6,
**dadurch gekennzeichnet,**
daß man dem Reaktionsgemisch ein Formiat von Alkali- und Erdalkalimetallen, Ammoniak oder Aminen in einem Molverhältnis von 0,1 bis 5:1, bezogen auf die eingesetzte Verbindung von Formel (II), zusetzt.

8. Verfahren nach einem der Ansprüche 1 bis 7,
**dadurch gekennzeichnet,**
daß man einen Katalysator auf Basis Palladium, Platin, Ruthenium oder Rhodium verwendet in einer Menge von 20 bis 2000 mg des reinen Edelmetalls pro kg eingesetzter Verbindung der Formel (II).

9. Verfahren nach einem der Ansprüche 1 bis 8,
**dadurch gekennzeichnet,**
daß man einen Katalysator auf einem Trägermaterial verwendet, dessen Edelmetall-Gehalt 0,1 bis 10 Gew.-% beträgt.

10. Verfahren nach Anspruch 9,
**dadurch gekennzeichnet,**
daß das Trägermaterial Aktivkohle ist.

11. Verfahren nach einem der Ansprüche 1 bis 10,
**dadurch gekennzeichnet,**
daß man die reduktive Formylierung bei einer Temperatur von 80 bis 140°C, vorzugsweise 100 bis 120°C, durchführt.

12. Verfahren nach einem der Ansprüche 1 bis 11,
**dadurch gekennzeichnet,**
daß man bei Verwendung von Formamid als Lösemittel die Cyclisierung durch Erhitzen im Temperaturbereich von 150 bis 190°C durchführt.

13. Verfahren nach einem der Ansprüche 1 bis 11,
**dadurch gekennzeichnet,**
daß man bei Verwendung von Ameisensäure als Lösemittel die Cyclisierung durch Erhitzen im Temperaturbereich von 100 bis 170°C und gegebenenfalls unter Druck bis 20 bar durchführt.

14. Verfahren nach einem der Ansprüche 1 bis 13,
**dadurch gekennzeichnet,**
daß man das gebildete Purin aus dem Reaktionsgemisch durch Eindampfen isoliert.

15. Verfahren nach einem der Ansprüche 1 bis 13,
**dadurch gekennzeichnet,**
daß das gebildete Purin durch Filtration, gegebenenfalls unter Zugabe von Wasser oder organischen Lösemitteln, aus dem Reaktionsgemisch isoliert wird.

16. Verfahren nach Anspruch 14 oder 15,
**dadurch gekennzeichnet,**
daß man das gebildete Purin in verdünnter Natronlauge löst, den Katalysator vom gelösten Produkt abfiltriert und das Purin mit Säure wieder ausfällt.

17. Verfahren nach Anspruch 16,
**dadurch gekennzeichnet,**
daß man vor der Fällung des Purins mit Säure mindestens eine Aktivkohlebehandlung der Lösung durchführt.
